# EUROPEAN PATENT APPLICATION

(11) **EP 1 057 460 A1**
(43) Date of publication of application: **06.12.2000**
(21) Application number: 00420007.7
(22) Date of filing: 18.01.2000
(51) Int. Cl.: A61F 2/24, A61F 2/06

(54) **Replacement valve assembly and method of implanting same**

(30) Priority: 11.10.1999 US 415810; 01.06.1999 US 137008 P
(71) Applicant: NUMED, INC., Nicholville, NY 12965-0129 (US)
(72) Inventor: Tower, Allen J., North Lawrence, NY 12965 (US); Bonhoeffer, Philippe, 75005 Paris (FR)
(74) Representative: Vuillermoz, Bruno

(57) **Abstract**

A replacement heart valve assembly (10) includes an intravascular radially expandable stent (14) and a biological valve (18) which is secured to the stent. The stent/valve assembly is positioned onto the inflatable portion (16) of a balloon catheter (20) and percutaneously implanted into a body lumen. The stent is made from a highly malleable material which allows localized plastic deformation and further allows subsequent radial reexpansion of the stent/valve assembly as needed. The biological valve is, for example, a harvested bovine jugular valve which can be adapted for use as a replacement for a malfunctioning human pulmonary valve.

## Description

### FIELD OF THE INVENTION

This invention relates generally to implantable intravascular devices, and more specifically to a replacement cardiac valve assembly that includes a biological valve which is attached to a radially intravascular stent, the assembly being capable of percutaneous implantation into a body lumen via balloon catheterization.

### BACKGROUND OF THE INVENTION

A persistent need exists in the field of medicine to be able to replace malfunctioning human heart valves during the life of a patient.

A number of advancements have been made both to the creation of replacement cardiac valves and to procedures for implanting replacement valve assemblies. For example, Cox (U.S. Patent No. 5,824,063) describes a method for replacing a heart valve using synthetic tubular material to selectively produce, for example, a bicuspid or tricuspid valve. Alternately, a method is described which utilizes biological material which has been harvested from the small intestine of the patient, or from a small animal or human cadaver. Each of the valves described by this patent are implanted by means of open cardiac surgery.

Bessler (U.S. Patent No. 5,855,601) describes the placement of an artificial heart valve onto a fairly rigid intravascular stent which can be delivered using a balloon catheter.

According to this patent, a stent made from titanium, titanium alloy, stainless steel or other similar rigid material in the form of a wire mesh receives a synthetic valve. The completed assembly is percutaneously and transluminally implantable to the lumen of interest.

Though the latter stent/valve assembly overcomes a number of problems by avoiding open heart surgery for initial implantation, this procedure is not considered optimal. First, the creation of an artificial cardiac valve is expensive and complex. Furthermore and despite significant engineering advances into synthetic materials and biomechanics, it is still commonly held that there is no true substitute for precisely replicating the opening and closing action demonstrated by so-called "natural" cardiac valves.

In addition, the above type of radially expandable stent, such as those described in U.S. Patent No. 4,733,665, is designed to be opened a single time and to a "final" and predetermined radial diameter. Therefore, if a need arises to reexpand the stent after implantation, as may be needed in small children whose vessels undergo growth changes, then open heart surgery would be necessary to remove the initially placed stent/valve assembly.

Furthermore, and because the entirety of the stent expands upon inflation, it is possible that improper alignment of the above described stent/valve assembly with the inflatable portion of the catheter could cause the stent/valve assembly to be dislodged into the heart itself upon inflation of the catheter, with death being a possibility.

### SUMMARY OF THE INVENTION

It is a primary object of the present invention to improve the state of the art of intravascular devices, and moreover to the state of replacement valve assemblies.

It is a further object of the present invention to provide a replacement valve assembly which can be implanted and subsequently adjusted without requiring open heart surgery.

It is a further object of the present invention to provide means for utilizing a biological "natural" valve as part of a replacement valve assembly which can be percutaneously implanted.

It is a further object to provide a replacement valve assembly which is designed to allow subsequent radial expansion, after implantation, as may be needed with young children given that growth changes are anticipated. Moreover, it is a further object to provide the capability of realizing such expansion, but without requiring open heart surgery.

Therefore, and according to a preferred aspect of the present invention, there is provided a replacement heart valve assembly comprising:
a radially expandable intravascular stent defined by at least one fine wire section formed into a cylindrical member and having an open-ended circular interior, at least one said fine wire section being made from a highly malleable material to allow localized plastic deformation of the stent, and
a biological valve attached to and disposed axially within the interior of said stent, wherein said stent can be disposed onto an inflatable portion of a balloon catheter and transluminally implanted.

According to a preferred embodiment, a plurality of cylindrical fine wire sections an be used, the wire sections being welded together to provide uniform radial expansion of portions of the stent.

Preferably, and according to a preferred embodiment, the biological valve is a valve harvested from the jugular vein of a bovine cadaver, the jugular valve being approximate in size to a human pulmonary valve.

Most preferably, the above biological valve includes a set of two or three circumferential leaflets disposed along an interior wall which expand as needed to allow blood flow in a first axial direction but prevent flow in a second opposite axial direction.

According to a preferred embodiment, the intravascular stent is made from a plurality of cylindrical fine wire sections, each being made from a highly malleable material which has been fully annealed to remove as much spring memory as possible. The material, therefore, can be plastically and locally deformed thereby allowing the stent to be expanded to a first radial diameter and if needed reexpanded, to a second or subsequent radial diameter which is larger than the first radial diameter. According to a preferred embodiment, wires consisting of a combination of platinum and iridium are used, though other malleable materials such as pure platinum and gold alloys, among others can be substituted.

Each of the cylindrical wire sections are axially disposed as part of a tubular configuration having an interior which includes a pair of open ends. Each cylindrical wire section is formed with a series of substantially sinusoidal bends which extend along the axial plane of the stent but do not protrude therefrom. More particularly, each of the sinusoidal bends includes a pair of sides, one of which is shared with an adjoining circumferentially disposed bend wherein adjacent wire segments are interconnected into a unitary structure by welding adjacent apices together. As such, the stent being made from a malleable material can easily and locally deform when a radial force is applied. The welded interconnection provides substantial expansion uniformity with regard to a specific wire section which is acted upon. This interconnection also greatly restricts the amount of axial shrinkage resulting when the stent is radially expanded, a problem commonly found in the stainless steel or tantalum type of stent described by Bessler and Palmaz. Other malleable stents which may be useful in the above described valve implantation are described in Applicants' U.S. Patent Nos. 5,389,106, 5,161,547, 5,217,483 and 5,868,783, among others.

An advantage of the replacement valve assembly of the present invention is that a "natural" valve can be effectively used and implanted without requiring open heart surgery.

Still another advantage of the present invention is that the described replacement valve assembly is at least as reliable while being far more inexpensive to manufacture to implant than previously known valve assemblies.

The described replacement valve assembly provides particular benefits for young patients such as infants or small children whose vessels will naturally grow in size. The malleable nature of stent material also reduces the chances that the stent may be driven from the balloon if misaligned. Furthermore, the use of radiopaque materials such as platinum facilitates tracking and placement.

These and other objects, features and advantages will become apparent from the following Detailed Description which should be read in conjunction with the accompanying drawings.

### BRIEF DESCRIPTION OF THE DRAWINGS

Fig. 1 is a perspective view of a replacement valve assembly in accordance with a preferred embodiment of the present invention;
Fig. 2 is a perspective view of a preferred stent used in the replacement valve assembly of Fig. 1;
Fig. 3 is a perspective view of the biological valve as partially attached to the stent of Fig. 2;
Fig. 4(a) is a schematic view of the interior of the biological valve used in the replacement valve assembly of Fig. 1;
Fig. 4(b) is a schematic end view of the biological valve of Fig. 4(a)
Fig. 5 is an end view of the replacement valve assembly of Fig. 1 as shown with the attached biological valve in an opened condition; and
Fig. 6 is an end view of the replacement valve assembly of Figs. 1 and 5 when the valve is in a closed position.

### DETAILED DESCRIPTION

The following description pertains to a preferred embodiment of a replacement valve assembly in accordance with the present invention. In particular, the embodiment relates to a replacement pulmonary heart valve assembly for a small child or infant. It will be readily apparent to those of sufficient skill in the field, however, that the inventive concepts described herein are applicable to a variety of patients and can be used in a number of separate procedures.

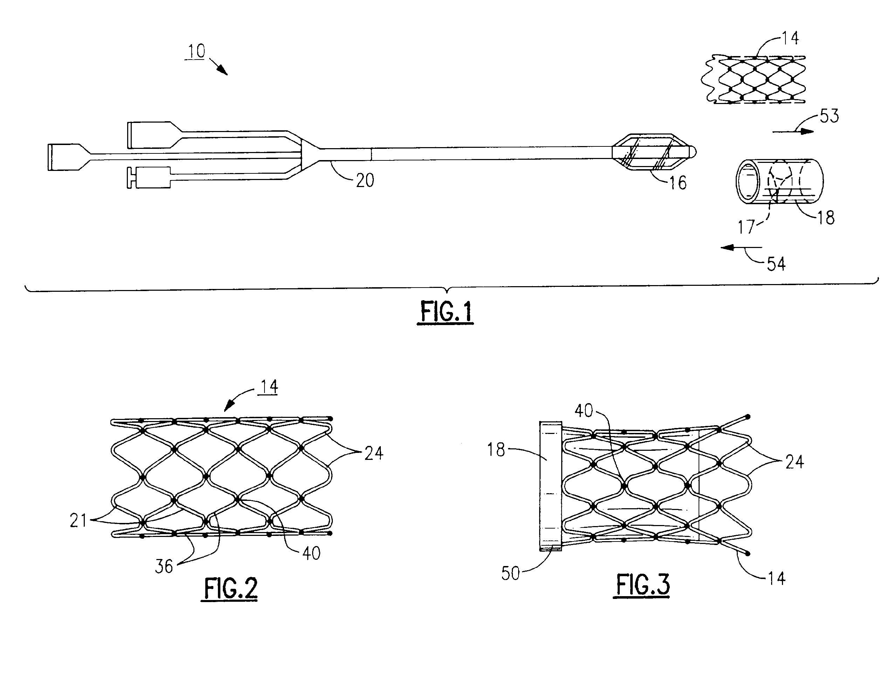

Turning to Fig. 1, a replacement valve assembly 10 according to this embodiment generally includes a radially expandable intravascular stent 14 and a biological valve 18. In brief, the biological valve 18 is sutured or otherwise attached to the intravascular stent 14 forming an unitary tubular structure which can be placed onto the inflatable portion 16 of a balloon catheter 20 and then transluminally implanted percutaneously into a blood vessel of interest. Details relating to each of the corresponding components of the replacement valve assembly 10 will herein be described.

First and referring to Fig. 2, the radially expandable intravascular stent 14 of this embodiment includes a plurality of cylindrical fine wire sections 21 which are interconnected into a single tubular structure. Each fine wire is made from a soft and highly malleable material which has been fully annealed to remove as much spring memory as possible. According to this embodiment, the material selected is a composition consisting of approximately 90% platinum and 10% iridium.

Prior to winding the fine wire sections 21 into a cylindrical shape, each individual fine wire section 21 is initially formed with a series of alternating substantially sinusoidally shaped bends 24. Though not shown but described more completely in commonly assigned and concurrently filed USSN 09/399,543, the stent 14 is initially formed by passing a first fine wire between a pair of spaced rows of cylindrical pins projecting from a top surface of a horizontal plate of an assembly fixture. The wire is wound along a serpentine path between adjacent rows of pins, thereby forming the series of substantially sinusoidally shaped bends 24 in a periodic alternating fashion. When completed, the resulting wire section 21 includes a predetermined number of sinusoidal bends 24 and a pair of free ends at opposing ends thereof. The above noted patent application is hereby incorporated by reference in its entirety.

As shown in Fig. 2, each of the substantially sinusoidal bends 24 is defined by a pair of sides 36, one of which is shared with an adjacent bend, which commonly converge into a common apex 40. As noted, the bends 24 alternate along the length of the wire section 21, and therefore each apex 40 is disposed on either lateral side thereof. A predetermined number of sinusoidal bends 24 are formed based on the number of rows of pins provided on the assembly fixture. According to the present embodiment, eight (8) bends are formed in the formed wire section 21.

Each adjacent fine wire section 21 is then subsequently axially interconnected by welding adjacent apices 40, thereby defining the overall axial length of the radially expandable stent 14.

Though not shown but described in USSN 09/399,543 patent application, a planar sheet of interconnected wire sections is removed from the assembly fixture and rolled onto a cylindrical mandrel (not shown) having a predetermined diameter. The opposing free ends of each of the interconnected individual wire sections 21 are then welded together completing a closed tubular wire frame or mesh-like structure having a circular cross section. When completed, the number of adjacently attached circumferential wire sections 21 define the overall axial length of the stent 14. In the present embodiment, seven (7) coupled wire sections 21 being used to define a structure which is approximately 1.5 inches in axial length. The overall axial length of the stent can easily be varied in accordance with the above; for example, stents of this type can vary between about 16 mm and about 150 mm.

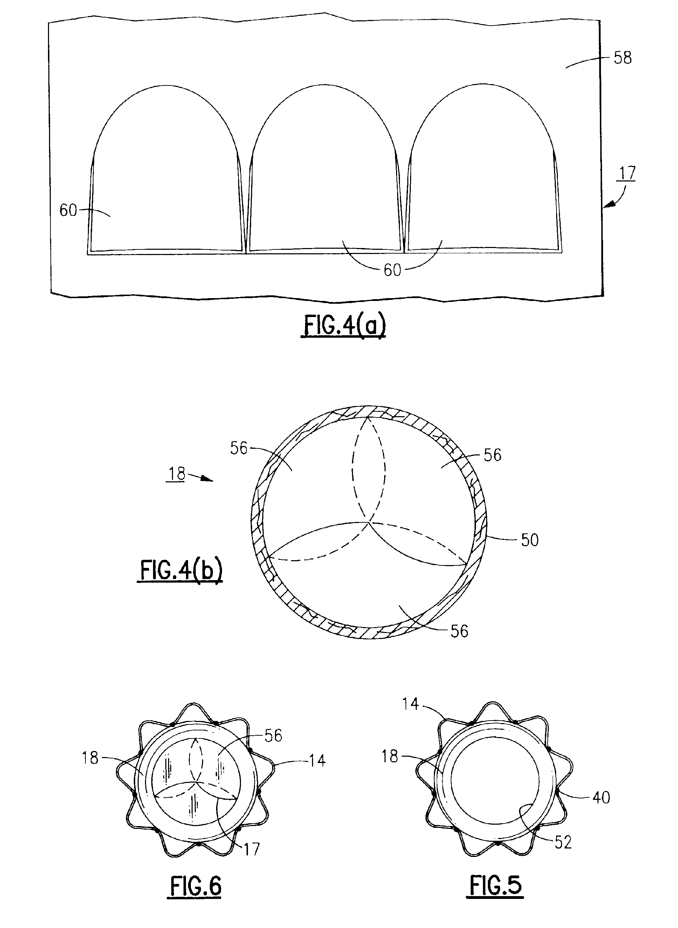

Description is now made relating to the biological valve 18 of this embodiment which is harvested from the jugular vein of a bovine cadaver. Referring to Figs. 3 , 4(a) and 4(b), the valve 18 includes an open ended tubular vein portion 50 having an interior 52 defined by a circumferential interior wall 58 including an interiorly disposed jugular vein valve 17. The jugular vein valve 17 itself is a tricuspid valve defined by three leaflets 56, each of which is formed along an equal portion of the circumference of the interior wall 58 of the tubular portion 50. Each of the leaflets 56 is defined by an extremely thin wall section 60 of elastic material extending around the perimeter thereof, the thin wall section depending from the interior wall 58 of the tubular portion 50. Each thin wall section 60 therefore has an arcuate shape which forms a pouch-like structure.

In operation and referring briefly to Figs. 1, 5 and 6, fluid passing through the tubular portion 50 in the direction 53 causes the pouch-like structure defined by each of the leaflets 56 to fill with fluid causing the flexible wall portion 60 of each of the leaflets 56 to elastically collapse inwardly toward the center of the opening 52 of the tubular portion 50. When filled, the expanded leaflets 56 fill the interior of the opening 52 of the tubular portion 56, thereby forming a highly effective valve closure. On the other hand, fluid flow in the reverse direction as indicated by arrow 54 is not restricted.

The size and thickness of the biological bovine material section, as initially harvested, is somewhat larger than that needed to permit provide an effective passageway when attached to the stent 14 to implant the assembly 10. Therefore, portions of the harvested section must first be trimmed in order to remove extraneous material. Sufficient material must remain, however, to allow the biological valve 18 to be fitted as an impermeable membranous layer for the mesh-like stent 14.

Prior to attaching the biological valve 13, the intravascular stent 14 is preexpanded to a suitable radial diameter which allows the biological sample to be sutured or otherwise attached to the interior of the stent 14. The stent 14 is attached to the inflatable portion 16 of a balloon catheter 20, such as that shown in Fig. 1, and the section is expanded by known means (not shown) to a predetermined radial diameter. Radial preexpansion of the stent 14 causes the sides of each sinusoidal bend 24 in contact with the inflatable portion 16 to widen circumferentially, therefore increasing the overall circumferential length of each corresponding fine wire section 21. However, and due in part to the welded interconnection between the wire sections 21, the stent 14 does not exhibit significant axial shrinkage.

The biological valve 18 is then sutured to the interior of the radially expandable intravascular stent 14, as shown partially in Fig. 3. Preferably, the tubular portion 50 will span the entire axial length of the stent 14. Prior to suturing, the biological section is tanned according to known methods which do not require further discussion.

Though not shown, the above replacement valve assembly 10 is then crimped onto the inflatable portion 16 of the balloon catheter 20. The malleable nature of the stent 14 and the flexible material of the biological valve 18 allows the entire replacement valve assembly 10 to be placed in a relatively low surface profile wherein neither the ends of the stent or the orientation of the bends 24 provide any sharp edges which could puncture an implanting balloon or the blood vessel through which the valve assembly 10 is implanted. The overall diameter of the inflatable portion of the catheter and the replacement valve assembly when fitted, according to this embodiment, is 16 French or smaller.

According to this embodiment and referring to Fig. 1, the inflatable portion 16 of the balloon catheter 20 includes respective inner balloon and outer balloons 42, 44 provided in overlapping relation to one another. Each of the balloons 42, 44 is made from a polyamide material which is biocompatible without producing an adverse reaction and expands to a predetermined radial diameter based on a specified inflation pressure.

Following attachment to the catheter 20, the replacement valve assembly 10 is percutaneously implanted after a guide wire (not shown) has first been guided to the area of interest, for example through the femoral artery. The replacement valve assembly 10 is then implanted over the guide wire, the position of which is easily tracked fluoroscopically using the radiopaque material of the stent.

Following alignment thereof, the stent 14 is initially positioned relative to the walls of the pulmonary artery by first inflating the inner balloon 42 from about 5 mm to 9 mm, which inflates the stent to the first radial diameter, but more importantly secures the stent 14 to the inflatable portion 16 . The outer balloon 44 is then inflated, additionally expanding the stent assembly to the second radial diameter (about 10 mm to 18 mm) such that the stent 14 is in supporting contact with the interior of the artery wall. In the meantime, the "old" valve is pushed against the side of the heart wall.

Each of the balloons 42, 44 are subsequently deflated and the catheter 20 is removed from the patient, leaving the replacement valve assembly 10 in position with the stent 14 in supporting contact with the interior wall of the artery.

As noted, implantation into a young child or infant means that the artery will grow in diameter over time. In that event, it may be necessary to effect radial expansion of the replacement valve assembly 10. Such expansion can easily be performed without requiring open heart surgery by locating the position of the replacement valve assembly 10, implanting a balloon catheter 20, Fig. 1, to the site of the replacement valve assembly, aligning the inflatable portion 16, such as that shown in Fig. 1, with the above assembly, and inflating the balloon so as to radially expand the replacement valve assembly, as needed. The malleable nature of the wire material of the stent 14 and the overall flexibility of the attached biological section 18 permit such radial expansion to occur. Subsequent expansion procedures can also be performed, provided the circumferential length of the cylindrical wire sections 21 of the stent 14 are initially sized correctly since this length form a limit to overall expansion of the assembly 10.

While the present invention has been particularly shown and described with reference to the preferred mode as illustrated in the drawing, it will be understood by one skilled in the art that various changes in detail may be effected therein without departing from the spirit and scope of the invention as defined by the claims. For example, it is contemplated that a similar introduction could be used to introduce a valve to the inferior vena cava to promote venous circulation, such as following pregnancy. Furthermore, other stents which are malleable in nature such as those described in U.S. Patent Nos. 5,389,106, 5,161,547, 5,217,483, and 5,868,783 can also support and expand a biological valve section, in the manner described above.

## Claims

1. A replacement heart valve assembly comprising:
a radially expandable intravascular stent defined by a plurality of wire sections formed into a cylindrical member and having a defined open-ended circular interior, each of said wire sections being made from a highly malleable material, said wire sections being welded together to provide uniform circumferential expansion; and
a biological valve sutured to said stent and axially disposed within the interior of said stent, wherein said stent can be positioned onto an inflatable portion of a balloon catheter and transluminally implanted.

2. A replacement heart valve assembly according to Claim 1, wherein said stent is made from a radiopaque material.

3. A replacement valve assembly according to Claim 1, wherein each of said cylindrical wire sections are made from annealed platinum having substantially all of its spring memory removed.

4. A replacement valve assembly according to Claim 1, wherein the wire sections forming said stent are made from a combination of 90 % platinum and 10% iridium.

5. A replacement valve assembly according to Claim 1, wherein each wire section is formed with a plurality of substantially sinusoidally shaped bends disposed along the circumferential length thereof.

6. A replacement valve assembly according to Claim 5, wherein each of said substantially sinusoidal shaped bends includes a pair of sides converging to an apex, said bends extending in a direction which is coaxial with a longitudinal axis of said stent, wherein apices of adjacent circumferential wire sections are welded together.

7. A replacement valve assembly according to Claim 1, wherein said biological valve includes a tubular vein segment having an interposed natural valve.

8. A replacement valve assembly according to Claim 7, wherein said tubular vein segment is taken from a bovine jugular vein.

9. A method for replacing heart valves, said method comprising the steps of:
securing a biological section of material to a radially expandable intravascular stent, said biological section including a tubular vein segment having a natural cardiac valve disposed within an interior thereof, said stent being made from fine wire segments, each made from a highly malleable material permitting local plastic deformation thereof and formed into a tubular configuration including an open-ended interior;
placing said stent having the secured biological section onto the inflatable portion of a balloon catheter;
percutaneously implanting said stent into a blood vessel of interest in relation to a diseased or malfunctioning valve;
inflating said balloon catheter to a predetermined radial diameter so as to place the stent into supporting contact with interior of the vessel wall; and
removing said catheter from said blood vessel.

10. The method of Claim 11, wherein said inflatable portion includes a first balloon and a second balloon overlapping said first balloon, said method including the steps of:
inflating said first balloon to a first inflation pressure to initially position the stent assembly onto said inflatable portion; and
inflating said second balloon to a second inflation pressure to secure said stent into the interior wall of the lumen.

11. The method of Claim 11, including the step of pre-expanding said stent prior to the securing step.

12. The method of Claim 11, wherein said biological section is a tubular section from a bovine jugular vein, said method including the step of implanting said stent and secured tubular section as a replacement for a human pulmonary valve.
